(19) Europäisches Patentamt
European Patent Office
Office européen des brevets

(11) **EP 1 987 774 A1**

(12) **EUROPÄISCHE PATENTANMELDUNG**

(43) Veröffentlichungstag:
**05.11.2008 Patentblatt 2008/45**

(51) Int Cl.:
***A61B 8/08*** *(2006.01)*

(21) Anmeldenummer: 07107418.1

(22) Anmeldetag: **03.05.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(71) Anmelder: **BrainLAB AG**
**85622 Feldkirchen (DE)**

(72) Erfinder:
• **Vollmer, Fritz**
 **80469, München (DE)**
• **Thiemann, Ingmar**
 **80686, München (DE)**

(74) Vertreter: **Schwabe, Hans-Georg**
**Patentanwälte Schwabe, Sandmair, Marx**
**Stuntzstrasse 16**
**81677 München (DE)**

(54) **Messung der Sonographie-Schallgeschwindigkeit mittels Markereinrichtung**

(57) Die vorliegende Anmeldung betrifft ein Verfahren zum Messen der Schallgeschwindigkeit des von einem Sonographiegerät ausgesendeten Ultraschalls in einer für Ultraschall durchlässigen Körperstruktur, wobei die von dem Sonographiegerät ausgehende Schallwelle durch die für Ultraschall durchlässige Körperstruktur wandert, bis die Schallwelle zumindest zum Teil an einem Reflexionsteil zum Sonographiegerät zurückreflektiert wird, um dort detektiert zu werden, wobei das Verfahren folgende Schritte aufweist:
Reflexions-Daten werden bereitgestellt, die Informationen über die relative Lage des Reflexionsteils zu einer Reflexions-Markereinrichtung umfassen;

Ort-Daten, die die relative Lage einer Sonographiegerät-Markereinrichtung zu einem Ausgangsort und einem Empfangsort der Ultraschallwellen beschreiben, werden bereitgestellt; Marker-Daten, die die relative Lage der Sonographiegerät-Markereinrichtung und der Reflexions-Markereinrichtung beschreiben, werden bereitgestellt;
Laufzeit-Daten, die die Laufzeit der Schallwelle vom Ausgangsort bis zum Empfangsort beschreiben, werden bereitgestellt;
aus den Reflexions-Daten, den Ort-Daten und den Marker-Daten wird die Weglänge berechnet, den die Schallwelle während der Laufzeit zurüelrlegt;
aus der berechneten Weglänge und den Laufzeit-Daten wird die Schallgeschwindigkeit bestimmt.

Figur 1

**Beschreibung**

Die vorliegende Erfindung betrifft die Messung der Schallgeschwindigkeit des Ultraschalls, der zur Sonographie (auch Echographie) verwendet wird. Insbesondere soll die Schallgeschwindigkeit in einer anatomischen Körperstruktur, in der eine Ausbreitung des Ultraschalls möglich ist, gemessen werden. Beispiele für derartige Körperstrukturen (eines Menschen oder Tieres) stellen insbesondere wasserhaltige Körperstrukturen, beispielsweise Weichteile, Gewebe, Leber, innere Organe, insbesondere Gehirn oder Extremitäten dar.

Bei der Sonographie wird zur Auswertung der Messdaten, insbesondere zur Skalierung eines Ultraschallbildes, eine Schallgeschwindigkeit zugrunde gelegt, die unabhängig von der spezifischen Körperstruktur für alle Körperstrukturen als gleich angenommen wird. Da die Schallgeschwindigkeit in die Bestimmung von mittels Sonographie bestimmten Abständen einfließt, kann eine nicht korrekte Annahme über die Schallgeschwindigkeit zu fehlerhaften Abstandsbestimmungen, insbesondere einer fehlerhaften Skalierung von Sonographiebildern führen.

Mit der Bestimmung der Schallgeschwindigkeit des Ultraschalls befassen sich folgende Druckschriften US 4,056,907; US 4,781,199; US 4, 669,482; und US 5,638,820.

Aufgabe der Erfindung ist es die Bestimmung der Schallgeschwindigkeit zu verbessern.

Vorstehende Aufgabe wird durch die Gegenstände der unabhängigen Ansprüche gelöst. Vorteilhafte Weiterbildungen gehen aus den abhängigen Ansprüchen hervor.

Erfindungsgemäß wird die Geschwindigkeit des Ultraschalls gemessen, wobei der Ultraschall von einem Sonographiegerät ausgesendet und detektiert wird. Das Sonographiegerät kann "einteilig" ausgebildet sein, d.h. einen kombinierten Sender und Empfänger aufweisen, insbesondere eine Ultraschallsonde, die Ultraschallwellen sendet und empfängt. Bei diesem, hierin als "einteilig" bezeichneten Sonographiegerät stimmen insbesondere der Ort, wo die Ultraschallwellen das Sonographiegerät verlassen und der Ort, wo diese vom Sonographiegerät zur Detektion empfangen werden, über ein oder die vorgenannten beiden Orte haben eine feste, vorbestimmte, insbesondere unveränderliche relative Lage zueinander. Letzteres kann beispielsweise der Fall sein, falls Sender und Detektor im Kopf einer Ultraschallsonde nebeneinander liegen. Auch betrifft die Erfindung ein Sonographiegerät, bei dem die vorgenannten beiden Orte nicht übereinstimmen und die relative Lage der beiden Orte zueinander veränderlich ist. Dieses Sonographiegerät wird hierin als "zweiteiliges" Sonographiegerät bezeichnet. Das zweiteilige Sonographiegerät umfasst einen ersten Ort zum Aussenden des Ultraschalls in einem ersten Teil des Sonographiegeräts (Ultraschallsender) und einen zweiten Ort, der vom ersten räumlich getrennt ist, zum Empfangen des Ultraschalls in einem zweiten Teil des Sonographiegerätes (Ultraschallempfänger), wobei beide Teile relativ zueinander beweglich sind, so dass die relative Lage zwischen den beiden Orten veränderlich ist. Insbesondere liegt die zu untersuchende Körperstruktur zwischen dem Ultraschallsender (erster Teil) und dem Ultraschallempfänger (zweiter Teil) des zweiteiligen Sonographiegeräts. Das hierin als einteilig bezeichnete Sonographiegerät arbeitet wie folgt: Es sendet Ultraschallwellen aus, die eine Körperstruktur durchlaufen, dann reflektiert werden, wiederum die Körperstruktur durchlaufen und dann von dem einteiligen Sonographiegerät (der Ultraschallsonde) detektiert werden. Die Reflektion kann an einem Teil der Körperstruktur (Reflexionsteil) erfolgen, der eine Ultraschallreflexion zur Folge hat. Ein Beispiel hierfür wäre eine Knochenstruktur. Die Reflexion ergibt sich durch die sich ändernden Ausbreitungseigenschaften des Ultraschalls, wenn die Ultraschallwelle das Reflexionsteil erreicht. Anders ausgedrückt, die Reflexion findet an der Grenzfläche zwischen der (in Ausbreitungsrichtung des Ultraschalls) vorgelagerten Körperstruktur und im Reflexionsteil statt. Soweit hierin von einer Reflexion an dem Reflexionsteil gesprochen wird, ist dabei die Reflexion an der vorgenannten Grenzfläche gemeint. Die Reflexion findet also statt, wenn die Ultraschallwelle einen Übergang zwischen zwei unterschiedlichen Materialien (mit unterschiedlichen Ultraschallausbreitungseigenschaften) erreicht. Ein Beispiel ist hierbei der Übergang vom Gewebe zum Knochen. Das Reflexionsteil sollte jedoch die Eigenschaft haben, das es nicht Ultraschall absorbiert, wie die beispielsweise bei luftgefüllten Hohlräumen (Darm) der Fall ist.

Die Reflexion kann an einer Knochenstruktur aber auch an einem körperfremden Teil erfolgen, beispielsweise an einer Platte, die Ultraschall reflektiert, wobei die zu untersuchende Körperstruktur zwischen der Platte und der Ultraschallsonde angeordnet ist. Die obigen Begriffe "einteilig" und "zweiteilig" schließen nicht aus, dass das Sonographiegerät mehr als ein oder zwei Teile umfasst, z.B. neben Sender und Empfänger auch einen Monitor und einen Computer.

Das erfindungsgemäße Verfahren umfasst insbesondere die folgenden Schritte: Vorzugsweise werden Daten bereitgestellt, die die Reflexion des Ultraschalls betreffen und die im Folgenden "Reflexions-Daten" genannt werden. Die Reflexions-Daten enthalten Information über die relative Lage des Reflexionsteils zu einer Reflexions-Markereinrichtung. Die Reflexions-Markereinrichtung ist eine Markereinrichtung, die eine feste räumliche Beziehung zu dem Reflexionsteil hat, also beispielsweise fest mit dem Reflexionsteil verbunden ist. Die Markereinrichtung kann aktiv oder passiv ausgebildet sein, d.h. Strahlen oder Wellen aktiv aussenden oder passiv reflektieren. Beispiele für Wellen oder Strahlen sind Licht, insbesondere Infrarotlicht oder Schallwellen. Die Markereinrichtung kann insbesondere reflektierende Kugeln umfassen, die in einer festen räumlichen Beziehung zueinander stehen.

Weiter werden vorzugsweise Daten bereitgestellt, die den Ort des Aussendens und Empfangens der Ultraschallwellen betreffen und im Folgenden "Ort-Daten" genannt werden. Die Ort-Daten beschreiben die relative Lage einer Sonographiegerät-Markereinrichtung zu einem Ausgangsort und einem Empfangsort der Ultraschallwellen. Die Sonographiegerät-Markereinrichtung ist eine Markereinrichtung (insbesondere wie oben beschrieben), die insbesondere eine feste relative Lage relativ zum Sonographiegerät hat, insbesondere mit dem Sonographiegerät fest verbunden ist. Insbesondere ist die relative Lage zwischen dem Ort des Aussendens der Ultraschallwellen von dem Sonographiegerät relativ zur Sonographie-Markereinrichtung fest. Insbesondere ist die relative Lage zwischen dem Empfangsort der Ultraschallwellen und der Sonographiegerät-Markereinrichtung fest.

Weiter werden vorzugsweise Daten bereitgestellt, die die Lage der Markereinrichtungen betreffen. Diese Daten werden hierin "Marker-Daten" genannt. Die Marker-Daten beschreiben die relative Lage der Sonographie-Markereinrichtung relativ zu der Reflexions-Markereinrichtung.

Vorteilhaft werden weiter Daten bereitgestellt, die die Laufzeit betreffen. Diese Daten werden "Laufzeit-Daten" genannt. Die Laufzeit-Daten beschreiben die Laufzeit der Schallwelle vom Ausgangsort bis zum Empfangsort.

Der Weg der Schallwelle vom Ausgangsort zum Empfangsort, insbesondere die Länge des Weges ist der Weg vom Ausgangsort zu dem Ort, wo die Ultraschallwelle reflektiert wird und von dort ausgehend zu dem Empfangsort. Diese von der Ultraschallwelle zurückgelegte Weglänge wird vorzugsweise basierend auf den Reflexions-Daten, den Ort-Daten und den Marker-Daten berechnet.

Ist die Weglänge bekannt, so lässt sich über die durch die Laufzeit-Daten beschriebene Laufzeit die Schallgeschwindigkeit des Ultraschalls bestimmen. Genauer handelt es sich hierbei um die mittlere Schallgeschwindigkeit, die die Schallwelle während der Laufzeit hat. Ist die für Ultraschall durchlässige Körperstruktur hinsichtlich der Schallgeschwindigkeit heterogen, so kann die bestimmte Schallgeschwindigkeit lokal von der tatsächlichen Schallgeschwindigkeit abweichen.

Das Reflexionsteil kann eine ausgedehnte Fläche aufweisen, so dass die entlang des vorgenannten Wegs laufenden Ultraschallwellen an verschiedenen Orten des Reflexionsteils potentiell reflektiert werden können. Um die Weglänge vom Ausgangsort der Ultraschallwellen zum Ort der Reflexion und von dort zum Empfangsort bestimmen zu können, wird vorzugsweise der Reflexionsort durch Informationen über eine Fläche des Reflexionsteils, an der eine Reflexion der Schallwellen stattfinden kann und Information über die Richtung der Ausbreitung der Schallwelle ausgehend vom Ausgangsort bestimmt. Der Schnittpunkt zwischen der von dem Ausgangsort in der bekannten Richtung entlang einer Ausbreitungslinie sich ausbreitenden Schallwelle und der Fläche des Reflexionsteils stellt somit den Reflexionsort dar.

Alternativ zu der vorgenannten Bestimmung des Ortes der Reflexion oder ergänzend zu der vorgenannten Bestimmung kann der Ort der Reflexion bei gegebener Fläche des Reflexionsteils auch nach dem Prinzip Einfallswinkel gleich dem Ausfallswinkel bestimmt werden, insbesondere wenn der Ausgangsort mit dem Empfangsort nicht übereinstimmt.

Befindet sich das Reflexionsteil außerhalb des Körpers eines Patienten, so lassen sich die Reflexions-Daten einfach z.B. durch Scannen, Abtasten oder optische Erfassung des Reflexionsteils ermitteln, um so die Lage des Reflexionsteil relativ zu der Reflexions-Markereinrichtung zu bestimmen. Befindet sich das Reflexionsteil im Inneren des Körpers eines Patienten, ist also beispielsweise eine Knochenstruktur, insbesondere ein Schädelknochen, so erfolgt die Bestimmung der Reflexions-Daten vorzugsweise mittels eines medizinischen Analyseverfahrens, das insbesondere Wellen oder Strahlen einsetzt, um insbesondere skalierte Information über die Lage bzw. räumliche Verteilung von Bestandteilen einer Körperstruktur zu gewinnen. Dabei werden insbesondere Röntgenanalyseverfahren (wie z.B. CT oder Röntgenaufnahmen oder Fluroskopiebilder, insbesondere aus diesem Röntgenanalyseverfahren rekonstruierte 3D Bilder) oder Kernspinaufnahmen (NMR, differenzielles NMR etc.) eingesetzt. Vorzugsweise erfolgt die Analyse bei am Körper des Patienten angebrachter Markereinrichtung. Dabei ist die Markereinrichtung vorzugsweise so gestaltet, dass sie sowohl durch das Analysegerät als auch durch eine Markerdetektionseinrichtung (z.B. Kamera oder Sensor, der auf reflektierte Wellen oder Strahlen anspricht) detektierbar ist. Beispielsweise werden Markerkugeln verwendet, die Licht reflektieren und einen im Röntgengerät nachweisbaren Metallkern enthalten. Die Markerkugeln sind vorzugsweise fest mit der Knochenstruktur verbunden. Beispielsweise ist eine Kugelhalterung in die Knochenstruktur eingeschraubt. Auf diese Art und Weise kann die relative Lage der Reflexions-Markereinrichtung relativ zu den Bestandteilen der Körperstruktur, insbesondere relativ zu einem Teil der Körperstruktur (z.B. Knochen) bestimmt werden, an dem potentiell die Schallwellen reflektiert werden. Die Information über die Lage der vorgenannten Fläche des Reflexionsteils umfasst insbesondere Informationen über Ausdehung und/oder Verlauf der Fläche. Insbesondere kann die Fläche (und deren Verlauf) durch eine mathematische Funktion beschrieben werden, um so den vorgenannten Schnittpunkt zwischen Ausbreitungslinie und Reflexionsfläche zu bestimmen.

Bei dem oben genannten medizinischen Analyseverfahren zum Bestimmen der Lage einer reflektierenden Körperstruktur, um die Reflexions-Daten zu bestimmen, wird vorzugsweise ein Analyseverfahren eingesetzt, bei dem es sich nicht um Sonographie handelt, also insbesondere keine Ultraschallwellen eingesetzt werden, so dass die gewonnenen Analysedaten nicht von der Schallgeschwindigkeit des Ultraschalls abhängen.

Wie bereits oben ausgeführt, kann das Sonographiegerät auch zweiteilig sein. Auch in diesem Fall kann der Weg der Ultraschallwellen vom Ausgangsort zum Empfangsort über ein Reflexionsteil verlaufen. Üblicherweise wird dies jedoch beim zweiteiligen Sonographiegerät nicht der Fall sein sondern der Ultraschall durchläuft ausgehend vom Ausgangsort die Körperstruktur und trifft dann auf das Empfangsteil des Sonographiegeräts. Im letzteren Fall ist der Weg durch den Abstand zwischen dem Ausgangsort und dem Empfangsort bestimmt. Im ersteren Fall errechnet er sich, wie oben bereits bezüglich des einteiligen Sonographiegeräts ausgeführt wurde.

Bei dem zweiteiligen Sonographiegerät gibt es insbesondere zwei separate Markereinrichtungen für das Sonographiegerät, nämlich eine Empfänger-Markereinrichtung, die eine feste relative Lage zum Empfangsort der Ultraschallwellen hat und insbesondere am Empfangsteil des Sonographiegeräts angebracht ist, und eine Sender-Markereinrichtung, die eine feste relative Lage zum Ausgangsort der Ultraschallwellen hat und insbesondere am Sendeteil des Sonographiegeräts angebracht ist. Durch Bestimmung der Lage der Sender-Markereinrichtung und der Empfänger-Markereinrichtung sowie basierend auf der vorgenannten bekannten relativen Lage zwischen Sender-Markereinrichtung und Ausgangsort und Empfänger-Markereinrichtung und Empfangsort lässt sich somit durch Detektion der Markereinrichtung der Abstand zwischen dem Ausgangsort und dem Empfangsort bestimmen. Damit ist auch die Weglänge bestimmt, die die Schallwelle zurücklegt.

Medizinische Analyseverfahren, insbesondere die bereits oben erwähnten, werden vorzugsweise eingesetzt, um Informationen über die mit der Sonographie zu untersuchenden oder untersuchten Körperstruktur zu gewinnen. Insbesondere werden diese medizinische Analyseverfahren vorzugsweise verwendet, um verschiedene Typen von Körperstrukturen im Falle einer heterogenen Körperstruktur zu identifizieren und insbesondere deren Ausdehnung und/oder Lage insbesondere relativ zu einer Markereinrichtung (z.B. Reflexions-Markereinrichtung) zu bestimmen.

Vorzugsweise wird weiter eine Datenbank bereitgestellt, die verschiedenen Typen von Teilen der Körperstruktur unterschiedliche Schallgeschwindigkeiten des Ultraschalls zuordnet. Die Schallgeschwindigkeit kann sich in unterschiedlichen Typen (beispielsweise Blut oder Gewebe) unterscheiden. Während beispielsweise mittels Kemspin die unterschiedlichen Typen von Körperstruktur erkannt und ihre Lage und Ausdehnung bestimmt, so kann eine Zuordnung der Schallgeschwindigkeit zu den einzelnen Typen erfolgen, so dass insbesondere ein räumliche Verteilung der Ultraschallgeschwindigkeit in der Körperstruktur bestimmt werden kann. Eine Kalibrierung dieser räumlichen Verteilung kann dann durch die erfindungsgemäße Bestimmung der Ultraschallgeschwindigkeit erfolgen. Die Kalibrierung kann so gestaltet werden, dass die mittlere Schallgeschwindigkeit durch die Körperstruktur der erfindungsgemäß bestimmten angepasst wird. Dies kann beispielsweise so erfolgen, dass eine (auf der Datenbank basierende) mittlere Schallgeschwindigkeit (Datenbank-Schallgeschwindigkeit) bestimmt wird, wie es sich aus den gespeicherten Daten und der medizinischen Analyse entlang des Schallweges geben würde. Dieser Wert wird mit der erfindungsgemäß bestimmten (gemessenen) Schallgeschwindigkeit verglichen, die einen Mittelwert entlang des Messweges darstellt. Die sich aus der Datenbank ergebenden Schallgeschwindigkeitswerte für die einzelnen Bereiche der Körperstruktur werden dann beispielsweise um einen bestimmten Prozentsatz angehoben oder abgesenkt, bis die mittlere Datenbank-Schallgeschwindigkeit mit der gemessenen mittleren Schallgeschwindigkeit übereinstimmt. Auf diese Art und Weise hat man eine kalibrierte räumliche Verteilung der Schallgeschwindigkeit in der Körperstruktur erzielt. Diese räumliche Verteilung kann dann verwendet werden, um die Skalierung des Ultraschallbildes weiter in ihrer Genauigkeit zu steigern.

Ein weiteres Beispiel für verschiedene Typen von Körperstrukturen sind gesundes und krankes Gewebe, also beispielsweise gesunde Hirnbereiche und ein Hirntumor. Berücksichtigt man die jeweils in den verschiedenen Körperstruktur-Typen geltende unterschiedliche Schallgeschwindigkeit und kalibriert diese dann entsprechend dem oben beschriebenen Verfahren anhand der Messung der mittleren Schallgeschwindigkeit, so kann die Genauigkeit der Skalierung weiter erhöht werden. Als medizinisches Analyseverfahren zur Typisierung der Körperstruktur und räumlich aufgelöste Messung und Lagebestimmung kann beispielsweise neben den oben beispielhaft genannten medizinischen Analyseverfahren insbesondere auch die Sonographie eingesetzt werden.

Die vorliegende Erfindung ist auch auf eine Vorrichtung gerichtet, die insbesondere eine Datenverarbeitungseinrichtung umfasst. Die Datenverarbeitungseinrichtung ist ausgebildet, die vorgenannten Daten in der vorgenannten Art und Weise zu verarbeiten. Insbesondere umfasst die Vorrichtung ein Eingabemittel zum Eingeben der zu verarbeitenden Daten. Weiter umfasst die Vorrichtung vorzugsweise ein Sonographiegerät, das insbesondere zur Ermittlung der Laufzeitdaten dient. Vorzugsweise ist weiter eine Detektionseinrichtung, beispielsweise eine Kamera vorgesehen, die ausgebildet ist, Signale zu messen.

Weiter kann die erfindungsgemäße Vorrichtung ein medizinisches Analysegerät umfassen, wie beispielsweise ein NMR-Gerät oder ein CT-Gerät, wobei die Messdaten an die Datenverarbeitungseinrichtung übermittelt werden.

Weiter ist die vorliegende Erfindung auf ein Programm gerichtet, das ein Verfahren gemäß der Erfindung durchführt, wenn es auf einem Computer läuft. Die von dem Programm zu verarbeitenden Daten werden dem Programm in üblicher Weise übermittelt. Die Daten können einen Computer beispielsweise über übliche Eingabemittel, wie Tastatur oder Schnittstellen zu Datenspeichern (z.B. Laufwerk für optische Disk) oder Datenbanken oder Internet übermittelt werden.

Bei der folgenden detaillierten Beschreibung werden weitere Vorteile und Merkmale der Erfindung offenbart. Die Figuren zeigen Folgendes:

Figur 1 zeigt die Ultraschallmessung an einem Gehirn.
Figur 2 zeigt das Prinzip der Ultraschallgeschwindigkeitsmessung.
Figur 3 zeigt die Messung bei einem zweiteiligen Sonographiegerät.
Figur 4 zeigt den Aufbau einer erfindungsgemäßen Vorrichtung.

Die vorliegende Erfindung ermöglicht insbesondere die Bestimmung der mittleren Schallgeschwindigkeit in einer Körperstruktur, z.B. im lebenden Gehirn oder in anderen Körperstrukturen. Die Körperstrukturen können homogen oder heterogen sein. Im Falle von heterogenen Körperstrukturen, wird vorzugsweise auf zusätzliches Datenmaterial von medizinischen Analyseeinrichtungen zurückgegriffen (siehe oben). Im Folgenden wird davon ausgegangen, dass es sich bei der zu untersuchenden Körperstruktur um ein homogenes Gewebe handelt. An dieses homogene Gewebe kann insbesondere ein anderer Typ einer Körperstruktur angrenzen, der sich hinsichtlich seiner Ultraschallausbreitungseigenschaften derart unterscheidet, dass dies zu einer Reflektion führt. Beispielsweise kann das Gewebe teilweise von einem Knochen umgeben sein, wie dies beim Gehirn der Fall ist.

Die Lage des Sonographiegerätes, insbesondere des Senders und Empfängers oder der Ultraschallsonde wird bevorzugt mittels eines Navigationsgeräts überwacht. Dazu sind vorzugsweise Markereinrichtungen, wie beispielsweise Referenzsterne an Sender, Empfänger und/oder Ultraschallsonde angebracht, so dass eine Navigation der Ultraschallsonde möglich ist, wie dies insbesondere bei IGS (Image Guided Surgery) üblich ist. Insbesondere ist es möglich mittels einer Detektionseinrichtung, die insbesondere Teil eines Navigationsgeräts ist, die Lage von Sender, Empfänger und/ oder Ultraschallsonde zu messen.

Die Ultraschall-Messung erfolgt beispielsweise im so genannten A-Modus oder B-Modus. Vorzugsweise werden mehrere Ultraschall-Scans durchgeführt, um die Genauigkeit der Messdaten zu erhöhen. Ergänzend zu der Ultraschallmessung wird vorzugsweise ein anderes medizinisches Analysegerät eingesetzt, um insbesondere die oben genannten Reflexions-Daten und/oder Körperstruktur-Daten zu bestimmen. Hierbei wird beispielsweise ein CT oder MRI eingesetzt. Die mittels des medizinischen Analysegeräts gewonnenen Daten werden vorzugsweise analysiert. Die Analyse kann manuell, beispielsweise von einem Arzt oder automatisch erfolgen. Die Analyse ist darauf gerichtet, Typen mit unterschiedlichen Ultraschalleigenschaften, insbesondere unterschiedlicher Echogenizität zu erkennen uns insbesondere deren Lage relativ zu Markern und/oder relativ zu homogenen, Ultraschall durchlässigen Gewebe zu bestimmen.

Erhält man einen Wert für die Schallgeschwindigkeit $c_s$ des Ultraschalls, so kann dieser verwendet werden, um die Ultraschalleinstellung des Sonographiegeräts zu korrigieren. Üblicherweise verwendet eine Sonographiegerät (Ultraschallgerät) einen festen Wert von 1540 m/s für $c_s$. Dieser Wert kann in der vorgenannten Art und Weise korrigiert werden, um insbesondere die Ultraschallbilder genauer zu skalieren und um Abstände mittels Ultraschall genauer bestimmen zu können. Insbesondere wird eine höhere Genauigkeit bei der navigierten Sonographie (Ultraschallabbildungstechnik) erzielt.

Üblicherweise nimmt man an, dass die Schallgeschwindigkeit des Ultraschalls in einem Gewebe einem mittleren Wert entspricht, wie er bei einem gesunden Gewebe oder einem Gewebe mit einer definierten Änderung (z.B. Fettleber) gegeben ist. Jedoch ändern einige Krankheiten die Ultraschallgeschwindigkeit, beispielsweise indem die Wassermenge im Gewebe erhöht wird (beispielsweise bei Zysten oder bei komprimierten Gewebe (z.B. hyperdichten Tumoren). Die z.B. durch geänderten Wassergehalt geänderte Schallgeschwindigkeit kann, soweit sie unbekannt ist, durch die vorliegende Erfindung gemessen werden und insbesondere kann eine Datenbank mit den so gemessenen Daten gefüllt werden, d.h. mit Daten für die Ultraschallgeschwindigkeit, die vom jeweiligen Typ der Körperstruktur abhängen und für diesen Typ spezifisch sind. Diese Datenbank kann dann in der oben erläuterten Weise zur Bestimmung einer räumlichen Vorteilung der Schallgeschwindigkeit in der untersuchten Körperstruktur z.B. mittels Bestimmung der Datenbank-Schallgeschwindigkeit herangezogen werden.

Insbesondere erlaubt die Erfindung In-vivo-Messungen der Schallgeschwindigkeit, z.B. beim Gehirn oder anderen Körperstrukturen eines Patienten. Wie oben ausgeführt wird, kann der im Stand der Technik fest vorgegebene Wert für die Schallgeschwindigkeit korrigiert werden, um die Genauigkeiten zu erhöhen. Somit lässt sich insbesondere die Genauigkeit bei der Navigation, z.B. eines Instruments oder eines Implantats erhöhen und die Skalierung und Längenmessung bei Ultraschallbildern verbessern.

Da die erfindungsgemäße Messung die Bestimmung der Ultraschallgesehwindigkeit erlaubt, kann auch so ein Rückschluss auf die Eigenschaften der Körperstruktur geschlossen werden. Wird beispielsweise eine vom gesunden Gewebe abweichende Schallgeschwindigkeit gemessen, so kann auf eine Krankheit geschlossen werden. Beispielsweise kann ein Tumor erkannt werden oder es können Änderungen in der Schallgeschwindigkeitseigenschaft des Tumors bestimmt werden, um so eine mögliche bösartige Veränderung des Tumors zu erkennen.

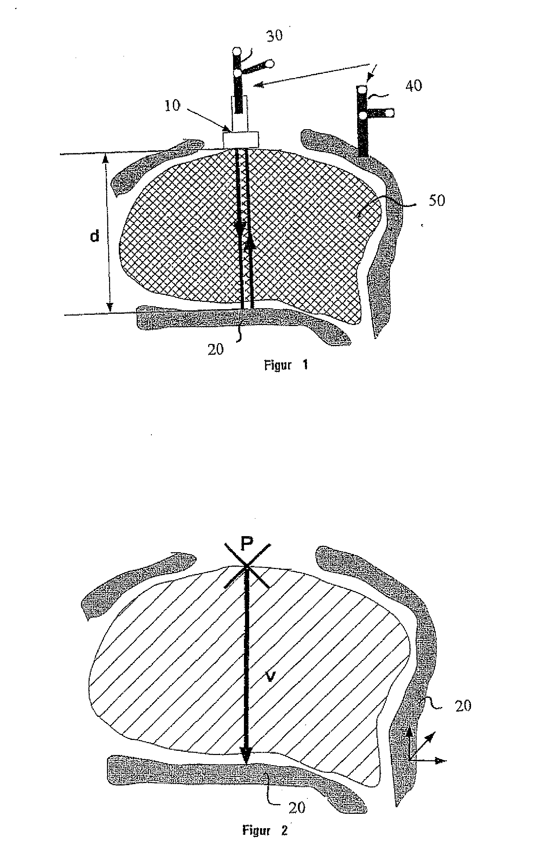

Das Prinzip der Ultraschallmessung ist wie folgt. Die mittlere Geschwindigkeit des Ultraschalls durch eine Körperstruktur ergibt sich aus der von dem Ultraschall zurückgelegten Wegstrecke geteilt durch die Zeit (Laufzeit), die das Signal benötigt, um die Wegstrecke zurückzulegen. Benötigt beispielsweise der Ultraschall die Zeit t, um von einer Ultraschallsonde 10 (siehe Figur 1) zu einer Knochenstruktur 20, die ein Beispiel für ein Reflexionsteil darstellt, zu gelangen und wird der Ultraschall von der Knochenstruktur 20 reflektiert und danach wiederum von der selben Ultraschallsonde 10 gemessen, so berechnet sich bei einem Abstand der Sonde zur reflektierenden Knochenstruktur d die Schallgeschwindigkeit $c_s$ wie folgt:

$$c_s = d/(t/2)$$

Um die Weglänge zu bestimmen, die der Ultraschall vom Sondenkopf bis zum Knochen zurücklegt, wird vorzugsweise ein Navigationssystem verwendet, das die Lage der Markereinrichtungen 30 und 40 detektiert. Zusätzlich werden Daten verwendet, aus denen sich die Lage der Knochenstruktur relativ zu der Markereinrichtung 40 ergibt. Vorzugsweise werden hierzu CT-Daten beispielsweise des Kopfes verwendet, wobei die Markereinrichtung 40 von der CT-Aufnahme erfasst wurde. Der Abstand zwischen dem reflektierenden Teil der Knochenstruktur 20 und dem Kopf der Ultraschallsonde 10 ist in der Figur 1 mit d bezeichnet. Die zwischen dem Kopf und dem reflektierenden Teil hin- und herlaufende Ultraschallwelle ist durch eine schwarze Linie mit Pfeilen angedeutet. Das Gehirn 50 ist schraffiert gezeichnet.

Die Lage des reflektierenden Teils der Knochenstruktur 20 kann von einem Arzt bestimmt werden und in die erfindungsgemäße Vorrichtung als Eingangsdaten eingegeben werden. Auch kann diese Lage automatisch bestimmt werden, indem die Richtung der Schallwellen aus der Lage der Ultraschallsonde bestimmt wird. An dem Ort, an dem sich eine Linie, die in diese Richtung verläuft, mit der Knochenstruktur schneidet, ist der Reflexionsort. Die relative Lage der Ultraschallsonde 10 relativ zu der Knochenstruktur 20 ergibt sich, wie oben ausgeführt ist, aus der Detektion der Markereinrichtungen 30 und 40. Auf diese Art und Weise lassen sich die Daten (Analyse-Daten) aus dem medizinischen Analyseverfahren (bei der Ultraschall nicht eingesetzt wird sondern z.B. Röntgen) mit den Ultraschalldaten abgleichen. Hierzu werden vorzugsweise Knochenstrukturen durch Segmentierung (Strukturerkennungsverfahren) im CT-Bild automatisch erkannt.

Vorzugsweise sind zumindest ein Teil der folgenden Umstände gegeben, um beispielsweise mittels Analysedaten (z.B. CT-Daten) die Ultraschallgeschwindigkeit insbesondere beim einteiligen Sonographiegerät zu bestimmen:

- Die Registrierung der Patientendaten (Analysedaten) in einem Bezugssystem (beispielsweise Bezugssystems eines Navigationssystems) ist möglich.
- Die medizinischen Analysedaten (Daten, die nicht mittels Ultraschall gewonnen wurden) ermöglichen eine Erkennung der knöchernen Körperstruktur, an der eine Reflexion von Ultraschallwellen stattfinden kann. Diese Erkennung kann automatisch oder manuell (z.B. durch den Arzt) erfolgen.
- Vorzugsweise ist das Gewebe zwischen dem Ultraschallkopf und dem Knochen homogen, so dass die bestimmte mittlere Ultraschallgeschwindigkeit aussagekräftig ist.
- Das mit Ultraschall untersuchte Gewebe ist von einer Knochenstruktur umgeben oder grenzt zumindest teilweise an eine Knochenstruktur an, so dass der Ultraschall zum Sondenkopf rückreflektiert werden kann.
- Der Abstand zum Knochen (oder zu einer sonstigen Struktur, insbesondere starre Struktur, an der ein Ultraschall reflektiert wird) kann von den Ultraschallwellen erreicht werden. Das heißt der Sondenkopf ist ausreichend dimensioniert, insbesondere hinsichtlich Leistung und Frequenz, so dass die Wellen an der reflektierenden Struktur ankommen und dort reflektiert werden können.

Vorzugsweise werden folgende Schritte vor einer Ultraschallmessung durchgeführt:

Die Knochenstrukturen werden identifiziert, insbesondere hinsichtlich ihrer Lage identifiziert und registriert bzw. zu den Ultraschallmessungen räumlich in Beziehung gebracht. Vorzugsweise wird bereits vor der Ultraschallmessung ein Bereich der reflektierenden Struktur festgelegt, an der die Reflexion tatsächlich stattfinden soll.

- Die Ultraschallsonde wird hinsichtlich ihrer Abmessungen erfasst, insbesondere wird die räumliche Beziehung zwischen dem Kopf der Ultraschallsonde, d.h. der Austrittsstelle der Ultraschallwellen und der Markereinrichtung (z.B. Referenzstern) bestimmt. Insbesondere wird die Ultraschallsonde im Bezugssystem eines Navigationssystems registriert.

- Der Patient wird im Bezugssystem, insbesondere im Bezugssystem des Navigationssystems und/oder der erfindungsgemäßen Vorrichtung registriert. Dazu wird insbesondere die Markereinrichtung 40 verwendet. Auf diese Art und Weise wird der Nicht-Ultraschalldatensatz (Analyse-Daten) relativ zu der Ultraschallsonde registriert. Damit wird mittels der Analyse-Daten auch die Lage des zuvor identifizierten reflektierenden Teils in die erfindungsgemäße Vorrichtung geladen und/oder bestimmt.

Vorzugsweise erfolgt die Messung zur Bestimmung der Ultraschallgeschwindigkeit im so genannten A-Modus der Ultraschallsonde. Nur ein einziger Schallimpuls wird entlang einer geraden Linie emittiert und der vom Knochen reflektierte Schallimpuls wird empfangen. Die Information kann auch im so genannten B-Modus gewonnen werden.

Wird der reflektierte Impuls empfangen, so ist dadurch die Laufzeit t der Schallpulswelle von der Ultraschallsonde zum Knochen und vom Knochen zurück zur Ultraschallsonde bestimmt. Insbesondere bei einem homogenen Gehirngewebe ist die Knochenkante sowohl im CT als auch im Ultraschall leicht detektierbar. Die Schallgeschwindigkeit v und die Richtung des Ultraschalls ausgehend vom Ausgangspunkt P der Ultraschallwellen ist in Figur 2 gezeigt. Ist ein kürzerer Weg zwischen einem möglichen Ausgangspunkt P und der Knochenstruktur 20 möglich, so kann dieser gewählt werden, um die Ultraschallgeschwindigkeit zu bestimmen, falls die Leistung des Ultraschallgeräts nicht ausreichend ist oder aus medizinischen Gründen nicht gewählt werden soll.

Aus der Detektion der Markereinrichtungen, die insbesondere mittels eines Navigationsgeräts durchgeführt wird, lässt sich somit die Spitze P der Ultraschallsonde und die Ausrichtung der Ultraschallsonde bestimmen. Aus der Ausrichtung der Ultraschallsonde gibt sich die Richtung der Geschwindigkeit v des Ultraschalls und damit auch die Richtung relativ zur Knochenstruktur. Hieraus kann, wie oben ausgeführt wurde, insbesondere die Position des reflektierenden Teils der Knochenstruktur bestimmt werden. Somit kann der Verlauf der Ausbreitung des Ultraschalls und insbesondere die Weglänge des Ultraschalls vom Ausgangsort zum Empfangsort der Ultraschallwellen bestimmt werden. Hierzu werden insbesondere skalierte und/oder hinsichtlich der Längendimensionen kalibrierte medizinische Analysedaten (CT-Daten) verwendet.

Ist die Weglänge bestimmt, so kann hieraus, wie oben ausgeführt wurde, mittels der Laufzeit die Ultraschallgeschwindigkeit berechnet werden. Vorzugsweise werden mehrere Messungen durchgeführt, um die Genauigkeit zu erhöhen und um insbesondere Registrierungsfehler (Fehler bei der Lagebestimmung der Ultraschallsonde) auszugleichen. Letzteres kann insbesondere dadurch erhöht werden, indem unterschiedliche Richtungen der Ausbreitung des Ultraschalls bei der Messung eingesetzt werden.

Alternativ oder zusätzlich können auch mehrere B-Modus-Ultraschallmessungen (Scans) verwendet werden. Dabei werden mehrere Verläufe (Wege oder Pfade) der Ultraschallwellen durch ein einziges Bild erfasst. Durch Anpassung (Skalierung) der Ultraschallwellen entsprechend an die Skalierung der medizinischen Daten, kann dann ebenfalls eine Skalierung des Ultraschallbildes und auch eine Messung der Ultraschallgeschwindigkeit durchgeführt werden.

Manche Ultraschallgeräte erlauben die Einstellung der Schallgeschwindigkeit. Bei diesen Geräten kann direkt, nach Messung der Schallgeschwindigkeit, dieses entsprechende Messergebnis am Gerät eingestellt werden, um genauerer Ultraschallbilder zu erzielen.

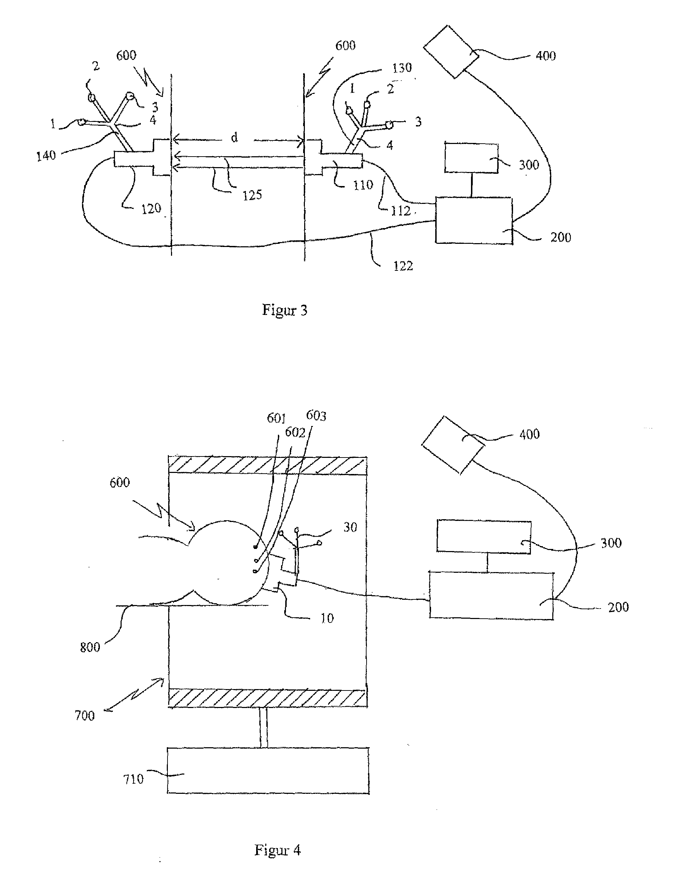

Figur 3 zeigt schematisch die Messung der Ultraschallgeschwindigkeit bei der Verwendung eines zweiteiligen Sonographiegeräts. Das Sonographiegerät umfasst einen Sender 110, einen Empfänger 120, eine Sendermarkereinrichtung 130, eine Empfängermarkereinrichtung 140. Die Markereinrichtungen 130 und 140 umfassen jeweils Markerkugeln 1, 2, 3, die durch einen Referenzstern 4 relativ zueinander bei feststehenden Positionen gehalten werden. Steuersignale und Detektionssignale von dem Ultraschallsender 110 und dem Ultraschallempfänger 120 werden über Leitungen 112 und 122 zu einem Auswertegerät, insbesondere Computer 200 übertragen. Die Daten können auf einem Monitor 300 angezeigt werden. Die Position der Markereinrichtungen 130 und 140 wird durch eine Detektionseinrichtung 400, insbesondere eine Kamera 400 detektiert. Die Detektionssignale werden ebenfalls an das Datenverarbeitungsgerät 200 übermittelt. Der Ultraschall breitet sich entlang der Linien 125 durch eine Körperstruktur 600, z.B. ein Bein, aus. Der Abstand zwischen dem Ultraschallsender und dem Ultraschallempfänger ist mit d bezeichnet. Die Ultraschallwellen 125 benötigen die Laufzeit t. Die Ultraschallgeschwindigkeit errechnet sich somit aus d geteilt durch t. Dabei errechnet sich die Laufzeit t als Differenz zwischen dem Zeitpunkt der Aussendung eines Ultraschallpulses von dem Sender 110 bis zum Empfang des Ultraschallpulses durch den Empfänger 120. Die vorgenannten Zeiten werden durch die Leitungen 112 und 122 übermittelt bzw. durch Steuersignale, die über diese Leitungen übertragen werden vorgegeben.

Figur 4 zeigt den Kopf eines Patienten 600. Im Kopf des Patienten sind Markerkugeln 601, 602 und 603 implantiert. Diese sind sowohl optisch durch eine Kamera 400 als auch durch ein CT-Gerät (C-Bogen) 700 nachweisbar. Der Patient 600 liegt auf einer Liege 800. Röntgenaufnahmen werden durch das Gerät 700 aus verschiedenen Richtungen gemacht, um ein dreidimensionales Röntgenbild zu erzielen. Die Ultraschallsonde 10 mit der Markereinrichtung 30 ist am Kopf des Patienten angebracht, so dass sich eine feste räumliche Beziehung zwischen Patientenkopf und Ultraschallsonde ergibt, die während der Messung konstant bleibt. Die Marker 601, 602 und 603 sowie die Markereinrichtung 30 werden durch die Detektionseinrichtung 400 gemessen. Die Detektionsdaten werden von der Datenverarbeitungseinrichtung 200 verarbeitet und können am Monitor 300 dargestellt werden. Die Datenverarbeitungseinrichtung 200 empfängt ebenfalls Daten von dem Datenverarbeitungsteil 710 des CT-Geräts. Somit kann durch Steuerung des Ultraschallsondengeräts und des CT-Geräts das Datenverarbeitungsgerät sämtliche Daten, insbesondere die Reflexions-Daten, die Ort-Daten, die Marker-Daten und die Laufzeit-Daten erhalten, um dann hieraus die Schallgeschwindigkeit zu messen. Vorzugsweise sind die Markerkugeln 601, 602 und 603 fest mit der Knochenstruktur des Patienten verbunden.

**Patentansprüche**

1. Verfahren zum Messen der Schallgeschwindigkeit des von einem Sonographiegerät (10) ausgesendeten Ultraschalls in einer für Ultraschall durchlässigen Körperstruktur (50),
wobei die von dem Sonographiegerät (10) ausgehende Schallwelle durch die für Ultraschall durchlässige Körperstruktur (50) wandert, bis die Schallwelle zumindest zum Teil an einem Reflexionsteil (20) zum Sonographiegerät (10) zurückreflektiert wird, um dort detektiert zu werden, wobei das Verfahren folgende Schritte aufweist:

   Reflexions-Daten werden bereitgestellt, die Informationen über die relative Lage des Reflexionsteils (20) zu einer Reflexions-Markereinrichtung (40) umfassen;
   Ort-Daten, die die relative Lage einer Sonographiegerät-Markereinrichtung (30) zu einem Ausgangsort (P) und einem Empfangsort (P) der Ultraschallwellen beschreiben, werden bereitgestellt;
   Marker-Daten, die die relative Lage der Sonographiegerät-Markereinrichtung (30) und der Reflexions-Markereinrichtung (40) beschreiben, werden bereitgestellt;
   Laufzeit-Daten (t), die die Laufzeit der Schallwelle vom Ausgangsort (P) bis zum Empfangsort (P) beschreiben, werden bereitgestellt;
   aus den Reflexions-Daten, den Ort-Daten und den Marker-Daten wird die Weglänge berechnet, den die Schallwelle während der Laufzeit (t) zurücklegt;
   aus der berechneten Weglänge und den Laufzeit-Daten (t) wird die Schallgeschwindigkeit (Cs) bestimmt.

2. Verfahren nach Anspruch 1, bei welchem der Ausgangsort (P) gleich dem Empfangsort (P) ist oder der Ausgangsort relativ zum Empfangsort eine feste, vorbestimmte Lagebeziehung hat.

3. Verfahren nach Anspruch 1 oder 2, bei welchem die Reflexions-Daten weiter Information über die Lage einer Fläche des Reflexionsteils (20) enthalten, an der eine Reflexion Schallwellen stattfinden kann, umfasst und die
Ort-Daten Information über die Richtung der Ausbreitung der Schallwellen umfassen und basierend auf den Informationen der Ort der Reflexion berechnet wird.

4. Verfahren nach einem der Ansprüche 1 bis 3, bei welchem der Reflexionsteil (20) ein Teil der Körperstruktur (20) ist, an dem Ultraschallwellen reflektiert werden.

5. Verfahren nach einem der Ansprüche 1 bis 3, bei welchem sich das Reflexionsteil außerhalb eines untersuchten Körpers eines Patienten befindet.

6. Verfahren nach Anspruch 4, bei welchem die Reflexions-Daten durch ein von der Sonographie abweichendes Analyseverfahren, insbesondere durch Einsatz Elektromagnetischer Strahlen oder -Wellen (CT, MRT) gewonnen werden.

7. Verfahren zum Messen der Schallgeschwindigkeit des von einem Sonographiegerät (110, 120) ausgesendeten Ultraschalls in einer für Ultraschall durchlässigen Körperstruktur,
wobei die von dem Sonographiegerät (110, 120) ausgehende Schallwelle durch die für Ultraschall durchlässige Körperstruktur (600) wandert, bis die Schallwelle vom Sonographiegerät (110, 120) empfangen wird, um detektiert zu werden, wobei das Verfahren folgende Schritte aufweist:

   Sender-Daten werden bereitgestellt, die die relative Lage zwischen einer Sender-Markereinrichtung (13) und einem Ausgangsort des Ultraschalls beschreiben;
   Empfänger-Daten, die die relative Lage zwischen einer Empfänger-Markereinrichtung (140) und einem Empfangsort des Ultraschalls beschreiben, werden bereitgestellt;
   Marker-Daten, die die relative Lage der Sender-Markereinrichtung (130) und der Empfänger-Markereinrichtung (140) beschreiben, werden bereitgestellt;
   Laufzeit-Daten, die die Laufzeit der Schallwelle vom Ausgangsort bis zum Empfangsort der Ultraschallwellen beschreiben, werden bereitgestellt;
   aus den Sender-Daten, den Empfänger-Daten und den Marker-Daten wird die Weglänge (d) berechnet, den die Schallwellen während der Laufzeit zurücklegen;
   aus der berechneten Weglänge und den Laufzeit-Daten wird die Schallgeschwindigkeit bestimmt.

8. Verfahren nach einem der Ansprüche 1 bis 7, bei welchem ein Ultraschallbild basierend auf der bestimmten Schallgeschwindigkeit skaliert wird.

9. Verfahren nach einem der Ansprüche 1 bis 8, bei welchem Körperstruktur-Daten, die Information über Lage und Typ unterschiedlicher Bereiche innerhalb der Körperstruktur bereitstellen, und Schallgeschwindigkeits-Daten, die eine Zuordnung von möglichen Typen einer Körperstruktur zu Schallgeschwindigkeiten umfassen, bereitgestellt werden, und wobei basierend auf den Körperstruktur-Daten und den Schallgeschwindigkeits-Daten den unterschiedlichen Bereichen jeweils Kandidaten-Werte für eine Schallgeschwindigkeit zugeordnet werden und die Kandidaten-Werte basierend auf der mittels des Sonographiegeräts bestimmten Schallgeschwindigkeit kalibriert werden.

10. Verfahren nach Anspruch 9, bei welchem zumindest ein Teil eines Ultraschallbild, das einen oder mehrere Bereiche unterschiedlichen Typs umfasst, basierend auf den kalibrierten Kandidaten-Werten skaliert wird.

11. Verfahren nach einem der vorhergehenden Ansprüche, bei welchem mehrere bestimmte Schallgeschwindigkeiten gemittelt werden, um die Genauigkeit zu erhöhen.

12. Vorrichtung zum Messen der Schallgeschwindigkeit des von einem Sonographiegerät (10) ausgesendeten Ultraschalls in einer für Ultraschall durchlässigen Körperstruktur (600),
wobei die von dem Sonographiegerät (10) ausgehende Schallwelle durch die für Ultraschall durchlässige Körperstruktur (600) wandert, bis die Schallwelle zumindest zum Teil an einem Reflexionsteil zum Sonographiegerät (10) zurückreflektiert wird, um dort detektiert zu werden, wobei die Vorrichtung folgendes umfasst:

das Sonographiegerät (10); und
eine Datenverarbeitungseinrichtung (200), die ausgebildet ist folgende Daten zu speichern und zu verarbeiten:

Reflexions-Daten, die Informationen über die relative Lage des Reflexionsteils (20) zu einer Reflexions-Markereinrichtung (601, 602,603; 40) umfassen;
Ort-Daten, die die relative Lage einer Sonographiegerät-Markereinrichtung (30) zu einem Ausgangsort und einem Empfangsort der Ultraschallwellen beschreiben;
Marker-Daten, die die relative Lage der Sonographiegerät-Markereinrichtung (30) und der Reflexions-Markereinrichtung (601, 602, 603; 40) beschreiben;
Laufzeit-Daten, die die mittels des Sonographiegeräts gemessene Laufzeit der Schallwelle vom Ausgangsort bis zum Empfangsort beschreiben;

wobei die Datenverarbeitungseinrichtung so ausgebildet ist, dass sie aus den Reflexions-Daten, den Ort-Daten und den Marker-Daten die Weglänge berechnet, den die Schallwelle während der Laufzeit zurücklegt, und
aus der berechneten Weglänge und den Laufzeit-Daten die Schallgeschwindigkeit bestimmt.

13. Vorrichtung nach Anspruch 12, die weiter umfasst:

eine Markerdetektionseinrichtung (400) zum Detektieren der Sonographiegerät-Markereinrichtung (30) und der Reflexions-Markereinrichtung (601, 602, 603; 40), um die Lage der Sonographiegerät-Markereinrichtung (30) und der Reflexions-Markereinrichtung (601, 602, 603; 40) zu bestimmen, um so die Marker-Daten zu bestimmen.

14. Vorrichtung zum Messen der Schallgeschwindigkeit des von einem Sonographiegerät (110, 120) ausgesendeten Ultraschalls in einer für Ultraschall durchlässigen Körperstruktur (600), wobei die von dem Sonographiegerät (110) ausgehende Schallwelle durch die für Ultraschall durchlässige Körperstruktur (600) wandert, bis die Schallwelle vom Sonographiegerät (120) empfangen wird, um detektiert zu werden, wobei die Vorrichtung folgendes aufweist:

das Sonographiegerät (110, 120);
eine Datenverarbeitungseinrichtung (200), die ausgebildet ist folgende Daten zu speichern und zu verarbeiten:

Sender-Daten, die die relative Lage zwischen einer Sender-Markereinrichtung (130) und einem Ausgangsort des Ultraschalls beschreiben;

Empfänger-Daten, die die relative Lage zwischen einer Empfänger-Markereinrichtung (140) und einem Empfangsort des Ultraschalls beschreiben;

Marker-Daten, die die relative Lage der Sender-Markereinrichtung (130) und der Empfänger-Markereinrichtung (140) beschreiben;
Laufzeit-Daten, die die mittels des Sonographiegeräts gemessene Laufzeit der Schallwelle vom Ausgangsort bis zum Empfangsort der Ultraschallwellen beschreiben;

wobei die Datenverarbeitungseinrichtung (200) so ausgebildet ist, dass sie aus den Sender-Daten, den Empfänger-Daten und den Marker-Daten die Weglänge berechnet, den die Schallwelle während der Laufzeit zurücklegen, und aus der berechneten Weglänge und den Laufzeit-Daten die Schallgeschwindigkeit bestimmt.

15. Vorrichtung nach Anspruch 14, die weiter umfasst:

eine Markerdetektionseinrichtung (400) zum Detektieren der Sender-Markereinrichtung (130) und der Empfänger-Markereinrichtung (140), um die Lage der Sender-Markereinrichtung und der Empfänger-Markereinrichtung zu bestimmen, um so die Marker-Daten zu bestimmen.

16. Programm, das, wenn es auf einem Computer läuft oder in einen Computer geladen wird, den Computer veranlasst, das Verfahren nach einem der Ansprüche 1 bis 11 auszuführen.

Figur 1

Figur 2

Figur 3

Figur 4

**EP 1 987 774 A1**

**Europäisches Patentamt**

**EUROPÄISCHER RECHERCHENBERICHT**

Nummer der Anmeldung

EP 07 10 7418

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 394 875 A (LEWIS JUDITH T [US] ET AL) 7. März 1995 (1995-03-07) <br> * Spalte 1, Zeile 25 - Zeile 32 * <br> * Spalte 3, Zeile 16 - Zeile 61 * <br> * Spalte 7, Zeile 19 - Zeile 21 * <br> * Spalte 9, Zeile 58 - Zeile 63 * <br> ----- | 1-16 | INV. <br> A61B8/08 |
| X | WO 2005/115246 A (AESCULAP AG & CO KG [DE]; UNIV ULM [DE]; KOZAK JOSEF [DE]; KEPPLER PET) 8. Dezember 2005 (2005-12-08) <br> * Abbildung 1 * <br> * Seite 1, Zeile 1 - Zeile 9 * <br> * Seite 3, Zeile 10 * <br> * Seite 7, Zeile 1 - Zeile 19 * <br> * Seite 8, Zeile 14 - Zeile 17 * <br> ----- | 1,7,12, 14 | |
| X | US 6 221 019 B1 (KANTOROVICH EDWARD [IL]) 24. April 2001 (2001-04-24) <br> * Spalte 3, Zeile 51 - Spalte 4, Zeile 7 * <br> * Spalte 11, Zeile 29 * <br> ----- | 1,7,12, 14 | |
| X | US 7 112 173 B1 (KANTOROVICH EDWARD [IL] ET AL) 26. September 2006 (2006-09-26) <br> * Spalte 3, Zeile 63 - Spalte 4, Zeile 19 * <br> * Spalte 20, Zeile 37 * <br> ----- | 1,7,12, 14 | RECHERCHIERTE SACHGEBIETE (IPC) <br><br> A61B |

Der vorliegende Recherchenbericht wurde für alle Patentansprüche erstellt

| Recherchenort | Abschlußdatum der Recherche | Prüfer |
|---|---|---|
| Den Haag | 6. September 2007 | Schwenke, Stephanie |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**
EP 07 10 7418

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-09-2007

| Im Recherchenbericht angeführtes Patentdokument | Datum der Veröffentlichung | Mitglied(er) der Patentfamilie | Datum der Veröffentlichung |
|---|---|---|---|
| US 5394875 A | 07-03-1995 | CA 2118532 A1<br>EP 0650075 A2<br>JP 7255723 A<br>JP 2004243140 A | 22-04-1995<br>26-04-1995<br>09-10-1995<br>02-09-2004 |
| WO 2005115246 A | 08-12-2005 | DE 102004026525 A1<br>EP 1758504 A1 | 22-12-2005<br>07-03-2007 |
| US 6221019 B1 | 24-04-2001 | KEINE | |
| US 7112173 B1 | 26-09-2006 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4056907 A
- US 4781199 A
- US 4669482 A
- US 5638820 A